# EUROPÄISCHE PATENTANMELDUNG

(11) **EP 2 918 252 A1**
(43) Veröffentlichungstag der Anmeldung: **16.09.2015**
(21) Anmeldenummer: 14158612.3
(22) Anmeldetag: 10.03.2014
(51) Int. Cl.: A61F 9/008

(54) **Vorrichtung zur Durchführung chirurgischer Behandlungen der Augenhornhaut**

(71) Anmelder: Schwind eye-tech-solutions GmbH & Co. KG, 63801 Kleinostheim (DE)
(72) Erfinder: Arba Mosquera, Samuel, 63739 Aschaffenburg (DE); Shraiki, Mario, 63811 Stockstadt (DE)
(74) Vertreter: Hofstetter, Schurack & Partner

(57) **Zusammenfassung**

Die vorliegende Erfindung betrifft eine Vorrichtung zur Durchführung chirurgischer Behandlungen des Auges, insbesondere der Augenhornhaut, umfassend eine Laservorrichtung die dazu eingerichtet ist, gepulste Behandlungsstrahlung mit einer Wellenlänge zwischen etwa 380 nm und 425 nm und einer Pulsdauer zwischen 0,1 ps und 10 ns zu emittieren, wobei die Pulsenergie der Behandlungsstrahlung zwischen 0,1 nJ und 5 µJ liegt.

## Beschreibung

Die Erfindung betrifft eine Vorrichtung zur Durchführung chirurgischer Behandlungen eines Auges, insbesondere der Augenhornhaut, umfassend eine Laservorrichtung die dazu eingerichtet ist, gepulste Behandlungsstrahlung einer vorbestimmten Wellenlänge abzugeben.

Derartige Vorrichtungen sind bekannt. So beschreibt die DE 101 48 783 A1 eine Anordnung zur nicht-invasiven optischen Bearbeitung von Geweben des Auges, insbesondere für die refraktive Corneachirurgie. Die Anordnung umfasst einen gepulsten Laser, der dazu geeignet ist, Strahlung in einem Wellenlängenbereich zwischen 500 nm und 1200 nm zu emittieren, wobei die Pulsdauer der Einzelpulse in der Größenordnung von Femtosekunden und die Energie des einzelnen Impulses in der Größenordnung von Nanojoule liegt. Nachteilig an dieser Anordnung und an dem damit verbundenen Verfahren zur Hornhautchirurgie ist jedoch, dass ab einer Wellenlänge von ca. 425 nm die Transmission verschiedener Komponenten des vorderen Augenabschnitts deutlich steigt. Somit kann ein überwiegender Anteil der eingesetzten Strahlung bzw. Strahlungsenergie zur Augenlinse und bis zur Netzhaut gelangen. Die Gefahr einer unbeabsichtigten Beschädigung dieser Augenkomponenten ist bei der Verwendung von Wellenlängenbereichen zwischen 500 nm und 1200 nm gegeben.

Die EP 1 787 607 B1 offenbart eine weitere Anordnung zur Durchführung chirurgischer Laserbehandlungen der Augenhornhaut. Dabei soll der Laser eine Behandlungsstrahlung mit einer Wellenlänge im nahen UV-Bereich zwischen 340 nm und 360 nm und einer Pulsdauer im Femtosekundenbereich emittieren, wobei die Pulsenergie der Behandlungsstrahlung zwischen 0,1 nJ und 5 µJ liegen soll. Nachteilig an dieser Anordnung ist jedoch, dass die Absorption des Hornhautepithels in dem vorgeschlagenen Wellenlängenbereich relativ stark ist. Dies bedeutet, dass entsprechend mehr Strahlungsenergie aufgewendet werden muss, um einen für eine Hornhautabtragung notwendigen Energiebetrag in den entsprechenden Bereichen der Hornhaut deponieren zu können. Damit steigt aber das Risiko einer Photokeratitis, d.h. einer Beschädigung der Hornhaut durch UV-Strahlung oder verschiedener Formen der Epitheliopathie deutlich an.

Aus der DE 10 2007 028 042 B3 ist eine Vorrichtung mit einem Laser zur Bearbeitung eines transparenten Materials durch eine im Bereich des Laserfokus erfolgende nichtlineare Absorption gepulster Laserstrahlung mit einer Wellenlänge in einem Bereich von 300 bis 1000 nm und einer Pulslänge in einem Bereich von 300 ps bis 20 ns, bekannt. Durch die Einbeziehung des genannten weiten Wellenlängenbereichs lassen sich auch bei dieser bekannten Vorrichtung, soweit sie im Bereiche der Behandlung des Auges zur Anwendung kommt, die im Vorhergehenden beschrieben Nachteile nicht mit Sicherheit verhindern.

Aufgabe der vorliegenden Erfindung ist es daher, eine Vorrichtung zur Durchführung chirurgischer Behandlungen eines Auges, insbesondere der Augenhornhaut, der eingangs genannten Art zu schaffen, welche sich durch eine erhöhte Energieeffizienz und eine verbesserte Sicherheit gegenüber unbeabsichtigten Schädigungen von Komponenten des Auges auszeichnet.

Diese Aufgabe wird erfindungsgemäß durch eine Vorrichtung mit den Merkmalen des Patentanspruchs 1 gelöst. Vorteilhafte Ausgestaltungen mit zweckmäßigen Weiterbildungen der Erfindung sind in den Unteransprüchen angegeben.

Eine erfindungsgemäße Vorrichtung zur Durchführung chirurgischer Behandlungen eines Auges, insbesondere der Augenhornhaut, umfasst eine Laservorrichtung die dazu eingerichtet ist, gepulste Behandlungsstrahlung mit einer Wellenlänge zwischen etwa 380 nm und 425 nm und einer Pulsdauer zwischen 0,1 ps und 10 ns zu emittieren, wobei die Pulsenergie der Behandlungsstrahlung zwischen 0,1 nJ und 5 µJ liegt. Es hat sich gezeigt, dass bei Verwendung der genannten Laserparameter die Gefahr von unbeabsichtigten Schädigungen von nicht zu behandelnden Komponenten des Auges deutlich reduziert wird. Insbesondere die Verwendung von Wellenlängenbereichen im sichtbaren Bereich sorgt für eine deutliche Verringerung der Absorption im Hornhautepithel. Dadurch verringert sich der Energieaufwand der notwendig ist, um einen für eine Hornhautabtragung notwendigen Energiebedarf in den zu behandelnden Bereichen der Hornhaut zu deponieren. Das Risiko einer Photokeratitis wird nahezu ausgeschlossen. Des Weiteren wird durch den erfindungsgemäß verwendeten Wellenlängenbereich von 380 nm bis 425 nm eine zu starke Transmission verschiedener Komponenten des vorderen Augenabschnitts ausgeschlossen. Ein überwiegender Anteil der eingesetzten Strahlung kann daher nicht bis zur Augenlinse oder bis zur Netzhaut gelangen, wodurch die Gefahr einer unbeabsichtigten Beschädigung dieser Augenkomponenten ebenfalls nahezu ausgeschlossen ist. Erfindungsgemäß kann die Pulsdauer vorteilhafterweise überwiegend im Pikosekundenbereich und die Pulsenergie der Behandlungsstrahlung zwischen 0,1 nJ und 5 µJ eingestellt werden. Die Vorteile der Verwendung von ultrakurzen Laserpulsen bei möglichst geringen Pulsenergien sind bekannt.

Insbesondere kann die Wellenlänge der Behandlungsstrahlung zwischen 380 und 405 nm liegen. Die Transmission der verschiedenen Komponenten des vorderen Augenabschnitts ist bei einer oberen Wellenlängengrenze von 405 nm nochmals geringer als bei 425 nm, wodurch sich für bestimmte chirurgische Anwendungen der Augenhornhaut oder anderer Komponenten des vorderen Augenabschnitts Vorteile ergeben. Des Weiteren konnte überraschenderweise festgestellt werden, dass bei einer Wellenlänge der gepulsten Behandlungsstrahlung von ungefähr 380 nm sich besonders gute Ergebnisse bei der Glaukombehandlung oder dem so genannten "Crosslinking" der Hornhaut ergeben. Hierbei wird beispielsweise Riboflavin als Zusatz für die korneale Kollagenvernetzung unter zusätzlicher Bestrahlung der Hornhaut verwendet. Die Pulsenergie kann dabei zwischen 0,1 nJ und 5 µJ variiert werden. Auch die Pulsdauer kann in dem erfindungsgemäßen Bereich zwischen 0,1 ps und 10 ns eingestellt werden. Besondere Vorteile ergeben sich zudem bei Pulsdauern zwischen 0,1 ps und 250 ps, da durch die Verwendung ultrakurzer Laserpulse geringere Anteile mechanischer Energie in die behandelnden Bereiche des Auges eingebracht werden. Des Weiteren hat sich als vorteilhaft erwiesen, eine Wellenlänge von ungefähr 383 nm zu verwenden. Durch die Variation der Pulsdauer können Photodisruptionen in unterschiedlichen Energiebereichen erzielt werden. So können im Pulsdauerbereich zwischen 0,1 ps und 10 ps Photodisruptionen, insbesondere im Augenhornhautbereich, erzielt werden, die eine sehr hohe Energiedichte beinhalten und ein so genanntes "High Density Plasma" erzeugen. Es entsteht ein so genanntes Plasmaleuchten, das bekanntermaßen mit der Schwelle für den so genannten optischen Durchbruch gleichgesetzt wird. Photodisruptionen mittels eines Plasmas mit sehr hoher Energiedichte können für einen tieferen und breiteren Abtrag zum Beispiel der Augenhornhaut verwendet werden. Es können Bereiche der Hornhaut als so genannte Klappen ("Flaps") oder Ringe geschnitten werden, zudem besteht die Möglichkeit in der Hornhaut Taschen auszubilden, die zum Beispiel mit künstlichen Linsen gefüllt werden können. Die erfindungsgemäße Vorrichtung mit den vorgenannten Parametern kann auch für die Keratoplastie verwendet werden. Insgesamt ist es auch möglich, neben der Wellenlänge der gepulsten Behandlungsstrahlung, die bei ca. 383 nm liegt, auch Wellenlängen im gesamten erfinderischen Bereich zwischen 380 und 425 nm zu verwenden. Bei der Bereitstellung der erfindungsgemäßen Vorrichtung mit einer Behandlungsstrahlung von ungefähr 383 nm, einem Pulsenergiebereich zwischen 0,1 nJ und 5 µJ und bei längeren Pulsdauern im Bereich von 1 ps bis 1 ns, insbesondere von 1 ps bis 250 ps, erfolgt eine Photodisruption im Übergangsbereich zwischen einer relativ hohen Energiedichte im Plasma und einer niedrigen Energiedichte im entstehenden Plasma ("Low Density Plasma"). Die Photodisruption erfolgt im Übergangsbereich zwischen einem leuchtenden zu einem nicht-leuchtenden Plasma. Aufgrund der etwas geringeren Energiedichte können Materialabtragungen schärfer definiert werden. Auch hierbei können wiederum Flaps, Ringe oder Taschen in der Augenhornhaut erzeugt werden. Eine erfindungsgemäße Vorrichtung mit den zuletzt genannten Laserparametern kann wiederum für die Keratoplastie verwendet werden. Wiederum kann neben einer Wellenlänge der Behandlungsstrahlung von ungefähr 383 nm auch der komplette erfindungsgemäße Wellenlängenbereich von 380 bis 425 nm verwendet werden. Schließlich kann bei einer gepulsten Behandlungsstrahlung mit einer Wellenlänge von etwa 383 nm, einer Pulsenergie der Behandlungsstrahlung zwischen 0,1 nJ und 5 µJ und Pulsdauern zwischen 0,1 ns und 10 ns, insbesondere 0,1 ns bis 0,25 ns, eine Photodisruption der zu behandelnden Hornhautbereiche mit einem Plasma niedriger Energiedichte, das heißt einem so genannten nicht-leuchtenden Plasma, erfolgen. Die Bereiche der Photodisruption können hierbei besonders exakt definiert werden. Wiederum können mit einer erfindungsgemäßen Vorrichtung mit den zuletzt genannten Laserparametern Flaps, Ringe und Taschen in der Augenhornhaut erzeugt werden. Die Vorrichtung kann auch für die Keratoplastie verwendet werden. Neben der Wellenlänge der Behandlungsstrahlung von 383 nm kann natürlich mit den genannten Bereichen der Pulsenergie der Behandlungsstrahlung zwischen 0,1 nJ und 5 µJ und dem Pulsdauerbereich zwischen 0,1 ns und 10 ns, insbesondere 0,1 ns und 0,25 ns, auch der gesamte erfindungsgemäße Wellenlängebereich zwischen 380 und 425 nm verwendet werden.

Des Weiteren hat sich als besonders vorteilhaft erwiesen, wenn die Wellenlänge der gepulsten Behandlungsstrahlung bei 405 nm liegt. Dabei kann die Pulsdauer der einzelnen Laserpulse wiederum in einem Bereich zwischen 0,1 ps und 10 ns, insbesondere zwischen 0,1 ps und 250 ps bei einer Pulsenergie zwischen 0,1 nJ und 5 µJ liegen. Eine derart erfindungsgemäß eingerichtete Laservorrichtung kann insbesondere bei der Photodisruption der Augenlinse oder auch bei Kataraktbehandlungen Verwendung finden. Neben der Wellenlänge von 405 nm kann natürlich der gesamte erfindungsgemäße Wellenlängebereich zwischen 380 und 425 nm verwendet werden. Besonders vorteilhaft hat sich die erfindungsgemäße Vorrichtung erwiesen, wenn die Laservorrichtung dazu eingerichtet ist, eine Wellenlänge der Behandlungsstrahlung von ungefähr 405 nm bei einer Pulsdauer von 0,1 ps bis 10 ps und einer Pulsenergie der Behandlungsstrahlung von 0,1 nJ bis 5 µJ zu verwenden. Eine derartige Einstellung ist insbesondere vorteilhaft bei der Presbyopiebehandlung wie auch wiederum bei der Photodisruption der Augenlinse. Auch hier kann neben der Wellenlänge von 405 nm natürlich der gesamte erfindungsgemäße Bereich zwischen 380 und 425 nm der Behandlungsstrahlung verwendet werden.

Der erfindungsgemäße Wellenlängenbereich zwischen etwa 380 nm und 425 nm bedeutet, dass folgende Wellenlängen bereitgestellt werden können: 380 nm, 381 nm, 382 nm, 383 nm, 384 nm, 385 nm, 386 nm, 387 nm, 388 nm, 389 nm, 390 nm, 391 nm, 392 nm, 393 nm, 394 nm, 395 nm, 396 nm, 397 nm, 398 nm, 399 nm, 400 nm, 401 nm, 402 nm, 403 nm, 404 nm, 405 nm, 406 nm, 407 nm, 408 nm, 409 nm, 410 nm, 411 nm, 412 nm, 413 nm, 414 nm, 415 nm, 416 nm, 417 nm, 418 nm, 419 nm, 420 nm, 421 nm, 422 nm, 423 nm, 424 nm, 425 nm. Unter etwa 380 nm sind auch Wellenlängen in einem Bereich zwischen 375 nm und 380 nm zu verstehen. Des Weiteren hat es sich als vorteilhaft erwiesen, wie im Vorhergehenden bereits zum Teil beschrieben, dass die Pulsdauer der Behandlungsstrahlung zwischen 0,1 ps und 250 ps, zwischen 1 ps und 250 ps, zwischen 0,1 ps und 10 ps oder zwischen 0,1 ns und 10 ns beträgt. Je nach Wellenlänge, Pulsenergie und auch der Art des Lasers sowie der Art der durchzuführenden chirurgischen Behandlung des Auges, insbesondere der Augenhornhaut, können die genannten Pulsdauerbereiche angewandt werden. Entsprechendes gilt für die Pulsrepititionsrate der Behandlungsstrahlung, die erfindungsgemäß wenigstens 10 kHz beträgt, vorzugsweise zwischen 100 kHz und 1 MHz liegt.

In weiteren vorteilhaften Ausgestaltungen der erfindungsgemäßen Vorrichtung ist die Laservorrichtung ein Festkörperlaser oder ein Mikrochip-Laser. Entscheidend ist, dass die verwendeten Lasertypen in der Lage sind, eine gepulste Behandlungsstrahlung im Wellenlängenbereich zwischen 380 und 425 nm zu emittieren. Beispielhaft ist hierfür ein Indiumgalliumnitrid-Laser genannt. Das Emittieren gepulster Behandlungsstrahlung im Wellenlängenbereich zwischen etwa 380 nm und 425 nm durch eine Laserquelle mit einer höheren Grundwellenlänge kann natürlich auch durch Frequenzverdoppelung, -verdreifachung, -vervierfachung, etc., aus erzeugt werden. Da es sich hierbei um grundsätzlich bekannte Verfahren und Vorrichtungen handelt, soll an dieser Stelle nicht näher darauf eingegangen werden.

In weiteren vorteilhaften Ausgestaltungen der erfindungsgemäßen Vorrichtung weist diese mindestens eine Fokussiervorrichtung zur Fokussierung der Behandlungsstrahlung auf oder in der Augenhornhaut und/oder mindestens eine Ablenkeinrichtung zur Ablenkung der Behandlungsstrahlung von einer Laserquelle zur Augenhornhaut auf. Durch die Fokussiervorrichtung ist es möglich, die Behandlungsstrahlung exakt zu positionieren.

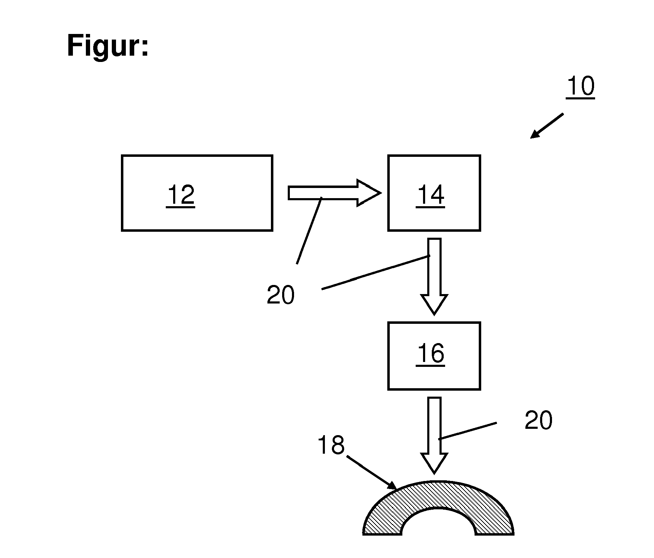

Weitere Merkmale der Erfindung ergeben sich aus den Ansprüchen, dem Ausführungsbeispiel sowie anhand der Zeichnung. Die vorstehend in der Beschreibung genannten Merkmale und Merkmalskombinationen sowie die nachfolgend in dem Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Dabei zeigt die Figur eine Prinzipdarstellung einer Vorrichtung 10 zur Durchführung chirurgischer Behandlungen eines Auges, insbesondere einer Augenhornhaut 18. Die Vorrichtung 10 umfasst dabei eine Laservorrichtung 12, die dazu eingerichtet ist, eine gepulste Behandlungsstrahlung 20 mit einer Wellenlänge zwischen etwa 380 nm und 425 nm und einer Pulsdauer zwischen 0,1 ps und 10 ns zu emittieren, wobei die Pulsenergie der Behandlungsstrahlung 20 zwischen 0,1 nJ und 5 µJ liegt. Man erkennt, dass die von der Laservorrichtung 12 emittierte Behandlungsstrahlung 20 über eine Ablenkeinrichtung 14 von der als Laserquelle dienenden Laservorrichtung 12 zur Augenhornhaut 18 geleitet wird. Nach der Ablenkeinrichtung 14 ist im Strahlengang eine Fokussiereinrichtung 16 zur Fokussierung der Behandlungsstrahlung 20 auf oder in der Augenhornhaut 18 angeordnet. Die so gesteuerten und fokussierten Pulse der Behandlungsstrahlung können dann zum Beispiel einen Flap oder eine Tasche in der Augenhornhaut 18 erzeugen. Aufgrund der relativ geringen Pulsenergien von 0,1 nJ bis 5 µJ können im Zusammenspiel mit der Pulsdauer von 0,1 ps bis 10 ns, insbesondere 0,1 ps bis 250 ps, und dem Wellenlängenbereich zwischen 380 nm und 425 nm der gepulsten Behandlungsstrahlung, insbesondere 380 nm und 405 nm, hervorragende Behandlungsergebnisse im Bereich der chirurgischen Augenbehandlung erzielt werden.

## Patentansprüche

1. Vorrichtung zur Durchführung chirurgischer Behandlungen eines Auges, insbesondere der Augenhornhaut (18), umfassend eine Laservorrichtung (12) die dazu eingerichtet ist, gepulste Behandlungsstrahlung mit einer Wellenlänge zwischen etwa 380 nm und 425 nm und einer Pulsdauer zwischen 0,1 ps und 10 ns zu emittieren, wobei die Pulsenergie der Behandlungsstrahlung zwischen 0,1 nJ und 5 µJ liegt.

2. Vorrichtung nach Anspruch 1, **dadurch gekennzeichnet, dass** die Wellenlänge der Behandlungsstrahlung zwischen 380 und 405 nm liegt.

3. Vorrichtung nach Anspruch 1 oder 2, **dadurch gekennzeichnet, dass** die Wellenlänge der Behandlungsstrahlung bei 380 nm, 383 nm oder 405 nm liegt.

4. Vorrichtung nach einem der vorhergehenden Ansprüche, **dadurch gekennzeichnet, dass** die Pulsdauer 0,1 ps bis 250 ps, 1 ps bis 250 ps, 0,1 ps bis 10 ps oder 0,1 ns bis 10 ns beträgt.

5. Vorrichtung nach einem der vorhergehenden Ansprüche, **dadurch gekennzeichnet, dass** die Pulsrepititionsrate der Behandlungsstrahlung wenigstens 10 kHz beträgt, vorzugsweise zwischen 100 kHz und 1 MHz liegt.

6. Vorrichtung nach einem der vorhergehenden Ansprüche, **dadurch gekennzeichnet, dass** die Laservorrichtung (12) ein Festkörperlaser oder ein Mikrochip-Laser ist.

7. Vorrichtung nach einem der vorhergehenden Ansprüche, **dadurch gekennzeichnet, dass** die Vorrichtung (10) mindestens eine Fokussiervorrichtung (16) zur Fokussierung der Behandlungsstrahlung auf oder in der Augenhornhaut (18) aufweist.

8. Vorrichtung nach einem der vorhergehenden Ansprüche, **dadurch gekennzeichnet, dass** die Vorrichtung (10) mindestens eine Ablenkeinrichtung (14) zur Ablenkung der Behandlungsstrahlung von einer Laserquelle zur Augenhornhaut (18) umfasst.
